# EUROPEAN PATENT APPLICATION

(11) **EP 1 212 980 A1**
(43) Date of publication of application: **12.06.2002**
(21) Application number: 01127567.4
(22) Date of filing: 19.11.2001
(51) Int. Cl.: A61B 5/0235

(54) **Flow control valve**

(30) Priority: 22.11.2000 JP 2000355137
(71) Applicant: Omron Corporation, Kyoto-shi, Kyoto 600-8530 (JP)
(72) Inventor: Fukui, Ryoichi, c/o Omron Corporation, Shimogyo-ku, Kyoto-shi, Kyoto 600-8530 (JP); Kurabayashi, Katsushi, c/o Omron Corporation, Shimogyo-ku, Kyoto-shi, Kyoto 600-8530 (JP)
(74) Representative: Kilian, Helmut, Dr.

(57) **Abstract**

A flow control valve has a housing (2) with a gas outlet (1a) and contains a mobile shaft (12) which can be moved towards or away from this gas outlet. A member (3) for closing and opening the gas outlet is attached to a front end of the mobile shaft. At least a portion of the front surface (31) of this member is formed as a curved surface such that when the mobile shaft is moved away from the gas outlet from the position where the gas outlet is completely closed by the closing member, a gap is formed more gradually between the gas outlet and the closing member such that a more accurate continuous control of the flow rate through the valve can be carried out.

## Description

### Background of the Invention

This invention relates to a flow control valve which may be used as an air discharging means, for example, for a hemadynamometer and more particularly to such a flow control valve capable of controlling air pressure to decrease gradually and slowly.

Among hemadynamometers of different kinds which have been proposed, there are those which function by increasing the pressure inside a cuff to a specified level and then lowering it gradually to measure the blood pressure of an individual during this course of pressure reducing process. Japanese Patent Publication Tokkai 6-47007 disclosed a flow control valve which may be used for reducing the pressure inside the cuff for such a hemadynamometer, characterized as comprising a pressure outlet for allowing compressed air to be discharged to the atmosphere and an orifice packing for opening and closing this pressure outlet and being structured such that each of end surfaces contacting the pressure outlet and the orifice packing is formed as a flat surface and that the end surface of the orifice packing is formed somewhat oblique to the surface perpendicular to the direction of motion of the driving shaft for moving the orifice packing.

Figs. 11 and 12 show an example of flow control valve thus structured with a housing formed with a frame case 2 and a bobbin 7. The frame case 2 is provided with a gas outlet 1a with a nozzle-like inner tube 1 opening inside and an external opening 2a which communicates with this gas outlet 1a through an inner space. Inside the housing, there is a mobile shaft 4 having a fixed shaft 12 penetrating therethrough disposed so as be able to move towards or away from the gas outlet 1a, and an orifice packing 3' is affixed to the tip of the mobile shaft facing the gas outlet 1a such that the gas outlet 1a can be opened and closed by moving the mobile shaft 4. The mobile shaft 4 is adapted to move to the left (with respect to Fig. 11A) by the electromagnetic force generated by two permanent magnets 5a and 5b and three electromagnetic coils 6a, 6b and 6c.

The mobile shaft 4 is connected to the frame case 2 through a damper 9 and is thereby biased in the direction to the right (again with reference to Fig. 11A). Both the front surface 31' of the orifice packing 3' and the opening surface of the gas outlet 1a are flat but they are not parallel to each other, the front surface 31' of the orifice packing 3' being oblique to the opening of the gas outlet 1a by a small angle, for example, of about 2.6°. As shown in Fig. 13, the orifice packing 3' is provided with a groove 30 for receiving therein the front end part of the mobile shaft 4.

The opening and closing of the gas outlet 1a by the orifice packing 3' shown in Figs. 11 and 12 will be explained next with reference to Fig. 14. Fig. 14A shows the gas outlet 1a completely closed by the orifice packing 3'. As the orifice packing 3' is retracted by a small distance of y from this completely closed condition, a gap of X1 is formed between the gas outlet 1a and the orifice packing 3', and a low-speed gas discharge condition is established, as shown in Fig. 14B. As the orifice packing 3' is further retracted, the gap becomes greater, as shown in Fig. 14C. As the orifice packing 3' is moved away from the gas outlet 1a and the gas outlet 1a becomes completely open, a high-speed gas discharge condition is established. In summary, Figs. 14A, 14B and 14C show the conditions under which the flow rate is being controlled.

By thus sloping the front surface 31' of the orifice packing 3' with respect to the gas outlet 1a, it is possible to control the discharge flow rate, and that especially when the flow rate is extremely small. In order to control the flow rate more accurately and continuously, however, it is desirable to modify the flow characteristic such that it will be expressed by a more gentle curve with respect to voltage changes when the flow rate is relatively small. With respect to Fig. 10 which shows the relationship between the flow rate and the voltage applied to control the position of the orifice packing, the range of voltage for controlling the flow rate (from the moment when the gas orifice 1a is completely open to the moment when it is completely closed) is indicated by 1e tter b in the case of the prior art flow control valve of Figs. 11 and 12. What is desired is to increase the range b such that a more accurate and continuous control of the flow rate will be possible.

In order to further increase the controllable voltage range b, however, the slope on the front surface 31' of the orifice packing 3' will have to be increased from 2.6°, say, to about 4°. If the slope of the front surface 31' is increased too much, however, it becomes necessary to accordingly increase the distance by which the orifice packing 3' must be moved towards the gas outlet 1a, and this further makes it necessary to increase the electromagnetic force. There is a structural limit, however, to how much this can be increased.

An attempt has been made to reduce the hardness of the material for the orifice packing 3', as well as to increase the slope of its front surface 31' but the improvement was only slight and no significant improvement could be accomplished. This was because the distance moved by the orifice packing 3' increases as its material is made softer and hence the discharge flow rate was not significantly affected by the change in the applied voltage.

### Summary of the Invention

It is therefore an object of this invention to provide a flow control valve capable of controlling the discharge flow rate more accurately and continuously especially when the discharge flow rate is relatively small.

A flow control valve embodying this invention, with which the above and other objects can be accomplished, may be characterized not only as comprising a housing having a gas outlet and an external outlet communicating with the gas outlet through an internal space, a mobile member, driving means for moving this mobile member towards or away from the gas outlet, and an outlet closing member such as an orifice packing which is attached to the mobile member and adapted to be pressed against and to close the gas outlet when the mobile member is pushed towards the gas outlet, but also wherein at least a portion of the front surface of the outlet closing member is curved, retracting away from the gas outlet.

Because at least a portion of the front surface of the outlet closing member is a curved surface, rather than a sloped flat surface, the area of the gap between the outlet closing member and the gas outlet changes more slowly and continuously when the gap is about to be created. As a result, a more accurate control on the flow rate can be accomplished.

### Brief Description of the Drawings

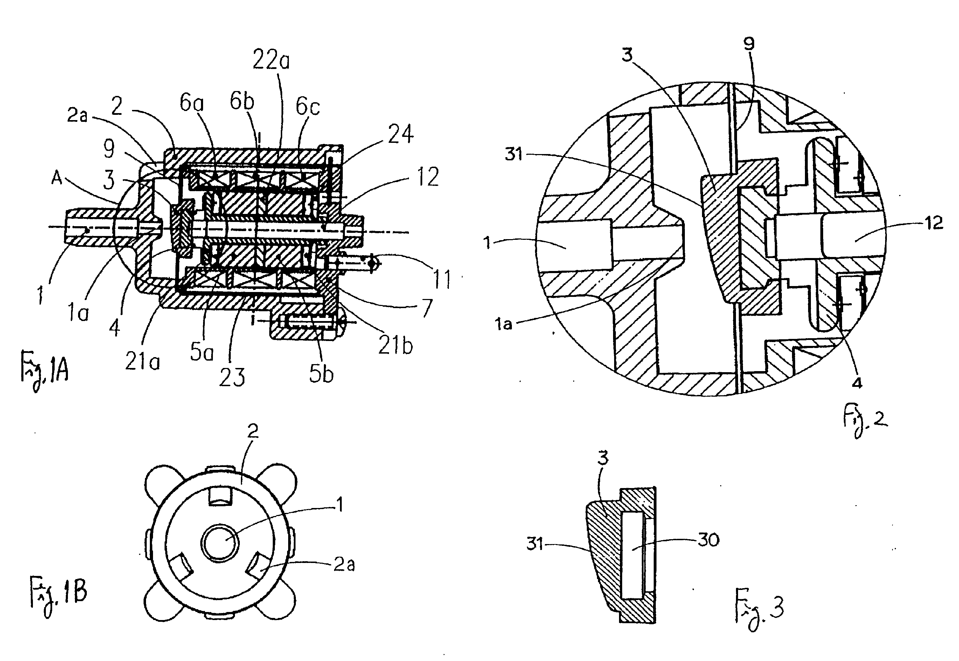

Figs. 1A and 1B, together referred to as Fig. 1, are respectively a sectional view and a left-hand side view of a flow control valve embodying this invention;

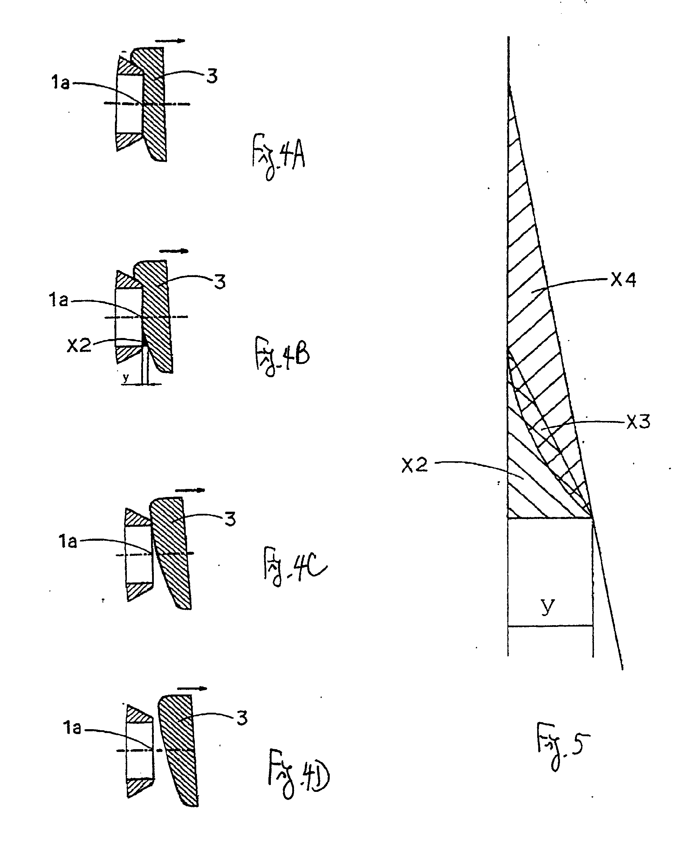

Fig. 2 is an enlarged view of a portion of the flow control valve shown in Fig. 1A;

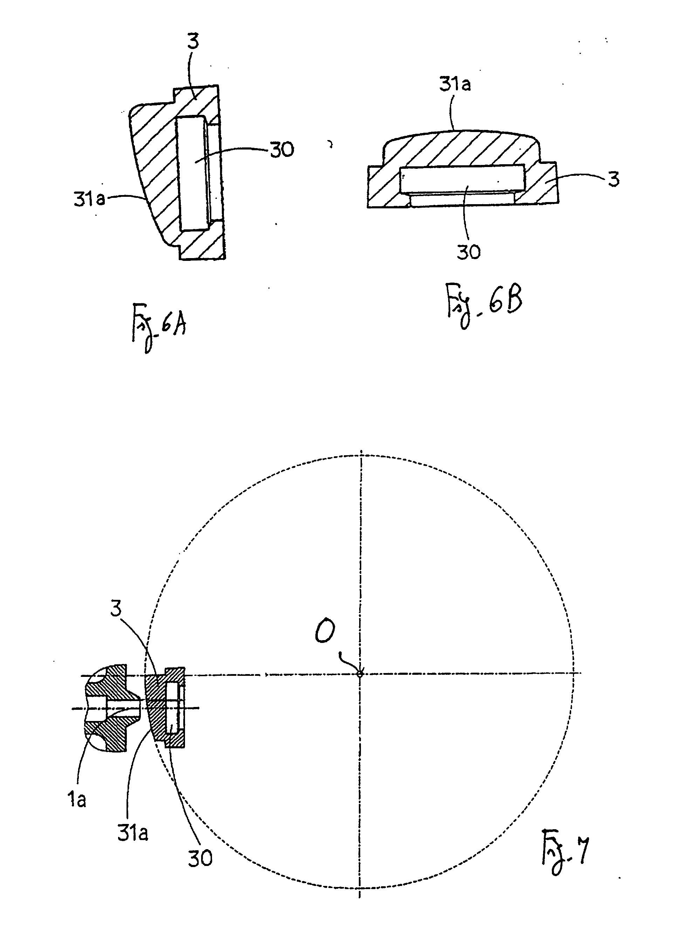

Fig. 3 is a sectional view of the orifice packing of Fig. 1;

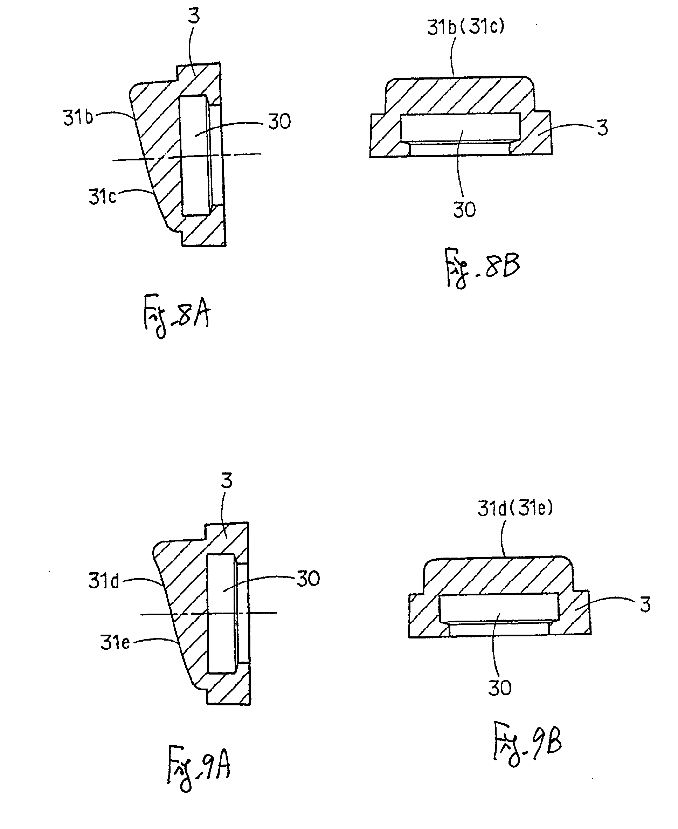

Figs. 4A, 4B, 4C and 4D show how the orifice packing functions in the flow control valve of Fig. 1;

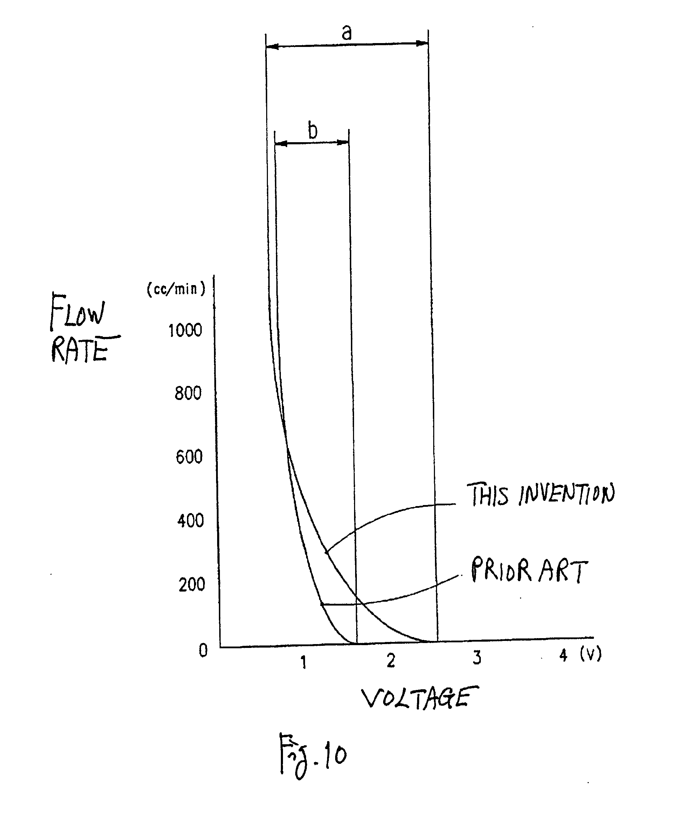

Fig. 5 shows the gap formed between the orifice packing and the gas outlet of the flow control valve of Fig. 1A;

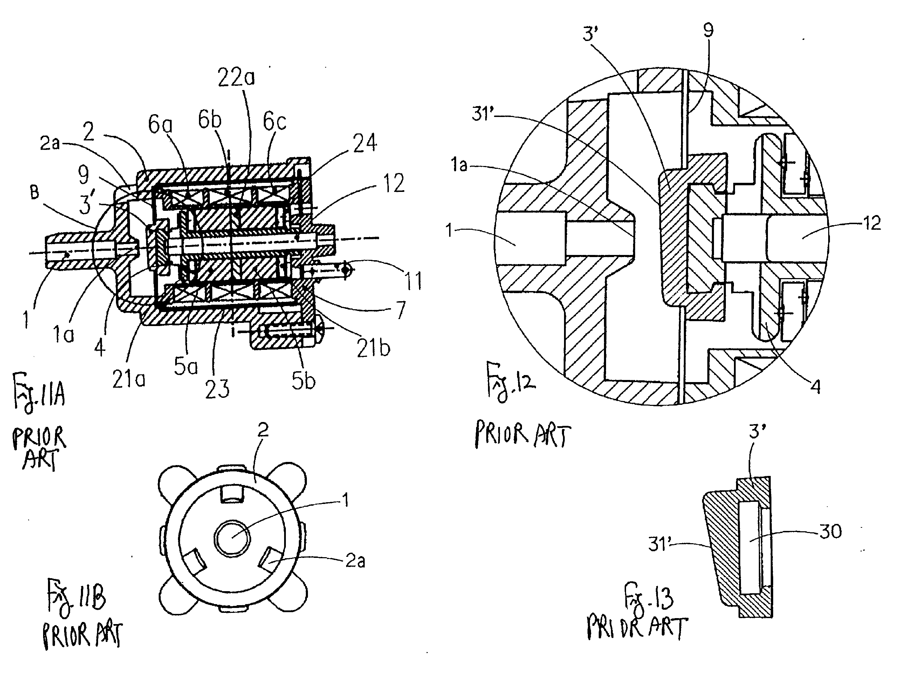

Figs. 6A and 6B are respectively a vertical sectional view and a horizontal sectional view of another orifice packing embodying this invention;

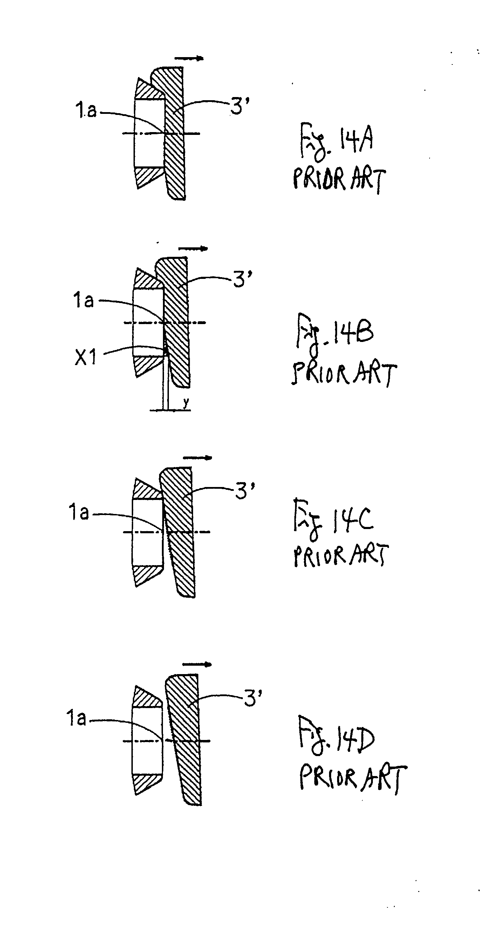

Fig. 7 is a drawing for explaining more in detail the shape of an orifice packing with a circularly arcuate front surface;
Figs. 8A and 8B are respectively a vertical sectional view and a horizontal sectional view of still another orifice packing embodying this invention;
Figs. 9A and 9B are respectively a vertical sectional view and a horizontal sectional view of still another orifice packing embodying this invention;
Fig. 10 is a graph of relationship between the voltage to push the mobile shaft of the flow control valve and the flow rate when flow control valves with orifice packing of different surface shapes are used;
Figs. 11A and 11B, together referred to as Fig. 11, are respectively a sectional view and a left-hand side view of a prior art flow control valve;
Fig. 12 is an enlarged view of a portion of the prior art flow control valve shown in Fig. 11A;
Fig. 13 is a sectional view of the orifice packing of Fig. 11; and
Fig. 14, consisting of Figs. 14A, 14B, 14C and 4D, shows how the orifice packing functions in the flow control valve of Fig. 11A.

Throughout herein, like or equivalent components are i ndicated by the same symbols without regards to whether they are components of the same of different flow control valve and may not necessarily be explained repetitiously.

### Detailed Description of the Invention

The invention is described next by way of an example with reference to Figs. 1 and 2, using the same numerals as used in Figs. 11 and 12 to indicate like or equivalent components. This flow control valve employs two permanent magnets 5a and 5b and three electromagnetic coils 6a, 6b and 6c for moving a mobile shaft 4. A housing is formed with a frame case 2 and a bobbin 7. The frame case 2 is provided with a gas outlet 1a with a nozzle-like inner tube 1 opening inside and a plurality (say, three) of external openings 2a which communicate with this gas outlet 1a through an inner space.

Inside the housing, there is a hollow mobile shaft 4 disposed so as to move towards or away from the gas outlet 1a. An orifice packing (or "the outlet closing member") 3 is affixed to the front tip of this mobile shaft 4 facing the gas opening 1a. The gas opening 1a is flat (and perpendicular to the axial direction of motion of the mobile shaft 4) but the orifice packing 3 has a front surface 31 which is curved in a circularly arcuate manner in the direction of retreating away from the gas outlet 1a. As shown in Fig. 3, the orifice packing 3 is provided with a groove 30 for receiving therein the front tip of the mobile shaft 4.

A fixed shaft 12 of a non-magnetic material penetrates the hollow interior of the mobile shaft 4 and is integrally affixed to the bobbin 7. The permanent magnets 5a and 5b, a yoke 22a and elastic members 21a and 21b are affixed to the external circumference of the mobile shaft 4 by means of a ring member 24 so as to move linearly together with the mobile shaft 4 as a single unit along the fixed shaft 12 within a specified range limited by the forward position where the orifice packing 3 contacts and completely closes the gas outlet 1a and the backward position where the rear end of the mobile shaft 4 hits the bobbin 7.

The permanent magnets 5a and 5b are disposed next to each other, sandwiching the yoke 22a in between, such that their same poles (such as the N poles) will be next to each other. The yoke 22a is excited by the poles of the two permanent magnets 5a and 5b and serves to most effectively utilize the magnetic forces of the magnets 5a and 5b.

The elastic member 21a disposed opposite an end surface of the magnet 5a is supported between the magnet 5a and the mobile shaft 4. The elastic member 21b disposed opposite an end surface of the magnet 5b is supported between the magnet 5b and the ring member 24.

Around the two permanent magnets 5a and 5b is the bobbin 7 provided with the three electromagnetic coils 6a, 6b and 6c. The middle coil 6b is somewhat longer than the other coils 6a and 6c in this example. The directions of winding of these coils 6a, 6b and 6c are determined such that the combined electromagnetic force of the magnets 5a and 5b and the electromagnetic coils 6a, 6b and 6c on the mobile shaft 4 will be in its axial direction of motion. Explained more in detail, the middle coil 6a is wound in one direction while the other coils 6a and 6c are wound in the opposite direction such that their directions of winding alternate and currents flow in opposite directions through each mutually adjacent pair of the coils all connected to an external terminal 11.

The two permanent magnets 5a and 5b are approximately symmetrically positioned with respect to the center plane of symmetry (shown by a broken line in Fig, 1A) of the middle coil 6b. A cylindrical yoke 23 surrounds the three coils 6a, 6b and 6c such that the coils 6a, 6b and 6c, the magnets 5a and 5b, the mobile shaft 4 and the fixed shaft 12 are all inside the cylindrical yoke 23. The mobile shaft 4 is connected to the frame case 2 through a damper 9 so as to be biased to the right-hand direction (with reference to Fig. 1) by the elastic force of the damper 9.

The fixed shaft 12 is a cylindrical bar, the mobile shaft 4 is a hollow cylinder, the magnets 5a and 5b, the yoke 22a, the elastic members 21a and 21b and the coils 6a, 6b and 6c are annular. Since they are coaxially disposed, the electromagnetic forces of the magnets 5a and 5b and the coils 6a, 6b and 6c can be most effectively usable as the compressive force by the mobile shaft 4. Since the fixed shaft 12 is of a non-magnetic material, it has no adverse effect on the electromagnetic forces.

Next, the operation of the flow control valve, thus structured, will be explained. To start, a current of a specified intensity is passed from the external terminal 11 to the coils 6a, 6b and 6c such that an electromagnetic force is generated by the coils 6a, 6b and 6c and the magnets 5a and 5b. As a result, the magnets 5a and 5b are subjected to a force in the left-hand direction. Explained in more detail, magnet 5a is electromagnetically pushed to the left by coils 6a and 6b, and magnet 5b is electromagnetically pushed to the left by coils 6b and 6c. These forces are together applied to the mobile shaft 4 to which the magnets 5a an 5b are affixed and overcome the biasing force of the damper 9 to push the mobile shaft 4 towards the gas outlet 1a. Thus, the orifice packing 3 contacts the gas outlet 1a and the inner tube 1 becomes completely closed, as shown in Fig. 4A.

After the inner tube 1 is closed, the current intensity to the coils 6a, 6b and 6c is gradually decreased. Since the generated electromagnetic force becomes gradually weaker accordingly, the force experienced by the magnets 5a and 5b becomes weaker. As a result of both the biasing force of the damper 9 and the elastic force of the material of the orifice packing 3, the mobile shaft 4 begins to move to the right gradually, becoming separated away from the gas outlet 1a.

When the mobile shaft 4 has retracted by a certain distance y, there appears a gap X2 between the gas outlet 1a and the orifice packing 3, as shown in Fig. 4B. A low-speed gas discharge condition is thereby established. As the mobile shaft 4 is further retracted away from the gas outlet 1a, the gas outlet 1a becomes completely open, as shown in Fig. 4C. As a large gap is formed between the gas outlet 1a and the orifice packing 3, as shown in Fig. 4D, there is established a high-speed gas discharge condition.

With reference to Fig. 4, the gas discharge flow rate is being controlled during the period between Figs. 4A and 4C. Since the front surface 31 of the orifice packing 3 is curved, the range in which the flow rate can be controlled is increased while the flow rate is relatively small. Explained more in detail, the gap X2 created when the mobile shaft 4 has been retracted by a distance y from the completely closed condition is smaller than the gap of X1 shown in Fig. 14B created on a prior art flow control valve. This comparison will be explained in detail with reference to Fig. 5.

In Fig. 5, X1 = X2+X3+X4 and it can be understood that the gap X2 in the case of the flow control valve according to the illustrated example is much smaller and hence a smaller control of the flow rate can be effected. Since the curvature of the surface varies smoothly, the flow rate can be controlled continuously. In other words, the flow rate can be controlled more accurately when the flow rate is relatively low according to the present invention. In Fig. 5, the portions X2 and X3 show when the slope of the front surface is changed. It should be kept in mind that a larger slope will require a stronger compressive force.

Figs. 6A and 6B show another orifice packing 3 embodying this invention characterized as having a spherically shaped front surface 31a. Fig. 7 shows more in detail how such a front surface 31a may be designed, indicating by letter O the position of the center of this spherically shaped front surface 31a. As shown in Fig. 7, the center O is required to be at a position higher than the gas outlet 1a by at least a specified vertical distance (on the condition that the orifice packing 3 moves horizontally towards and away from the gas outlet 1a). This requirement for a specified minimum vertical is imposed such that the gas outlet 1a will open gradually from one side only even if the center lines of the gas outlet 1a is somewhat displaced from the center of the orifice packing 3. In general, including situations where the motion of the orifice packing 3 towards and away from the gas outlet 1a is not horizontal, the requirement on the position of the center O may be expressed as being outside the cylindrical surface that would result if straight lines in the direction of motion of the orifice packing 3 are drawn from the circumference of the gas outlet 1a.

Many modifications and variations of the examples described above are intended to be within the scope of this invention. For example, Figs. 8A and 8B show still another orifice packing 3 embodying this invention, characterized as having a front surface with a flat portion 31b and a curved portion 31c. Figs. 9A and 9B show still another orifice packing 3 embodying this invention, characterized as having a front surface with a first portion 31d and a second portion 31e, the first portion 31d being a portion of a circle with its center on the side of the gas outlet 1a and the second portion 31e being a portion of another circle with its center on the side of the packing 3, the boundary of the two portions 31d and 31e being at the center of the packing 3.

In all these examples, the controllable portion of the range of voltage to be applied to the coils becomes greater (indicated by letter a in Fig. 10) than if the front surface of the packing is uniformly flat and hence a more accurate and continuous control of the flow rate is possible according to this invention.

## Claims

1. A flow control valve comprising:
a housing having a gas outlet and an external outlet communicating with said gas outlet through an internal space, said gas outlet defining a center outlet line;
a mobile member which is movable along said center outlet line towards and away from said gas outlet inside said housing;
driving means for moving said mobile member; and
an outlet closing member with a front surface, said output closing member being attached to said mobile member and adapted to be pressed against and to close said gas outlet when said driving means moves said mobile member towards said gas outlet,
wherein at least a portion of said front surface on one side of said a plane containing said center outlet line is a curved surface retracting away from said gas outlet.

2. The flow control valve of claim 1 wherein said front surface is a circularly arcuate curved surface.

3. The flow control valve of claim 2 wherein said circularly arcuate curved surface has a center located at a position outside a cylindrical surface formed by straight lines which are parallel to said center outlet line and pass through the circumference of said gas outlet.
